# EUROPEAN PATENT APPLICATION

(11) **EP 4 729 932 A1**
(43) Date of publication of application: **22.04.2026**
(21) Application number: 25208853.9
(22) Date of filing: 15.10.2025
(51) Int. Cl.: G01N 23/04, G01N 23/083, G01N 23/18, G01N 33/12

(54) **X-RAY INSPECTION DEVICE**

(30) Priority: 17.10.2024 JP 2024182038
(71) Applicant: Ishida Co., Ltd., Kyoto-shi Kyoto 606-8392 (JP)
(72) Inventor: YURUGI, Futoshi, Ritto-shi, Shiga, 520-3026 (JP); SUGIMOTO, Kazuyuki, Ritto-shi, Shiga, 520-3026 (JP); MASAOKA, Yunosuke, Ritto-shi, Shiga, 520-3026 (JP)
(74) Representative: Global IP Europe Patentanwaltskanzlei

(57) **Abstract**

To provide an X-ray inspection device that can inspect articles without having to provide additional X-ray line sensors even when the articles are conveyed by a plurality of conveyors having different conveying speeds.

In an X-ray inspection device 10, an X-ray line sensor 14 detects amounts of X-rays transmitted through a first article G1 and a second article G2 at a timing based on a conveying speed of a first conveyor 121. Speeds of the first conveyor 121 and a second conveyor 122 are known, and any effect of a speed difference on an inspection of the second article G2 is therefore preferably corrected. Therefore, there is no need for any additional X-ray line sensors 14 to be provided.

## Description

### TECHNICAL FIELD

The present invention relates to an X-ray inspection device.

### BACKGROUND ART

Conventionally, devices that inspect articles (such as raw meat) conveyed on a conveyor by irradiating the articles with X-rays and detecting the amount of X-rays transmitted through the articles with an X-ray line sensor have been widely known as X-ray inspection devices.

For example, in the X-ray inspection device disclosed in Patent Literature 1 (JP 2003-139723 A), articles lined up in parallel in a width direction of a conveyor are conveyed at the same speed, and the amount of X-rays transmitted through the articles can therefore be detected by a single X-ray line sensor at a timing based on a conveying speed of the conveyor.

### SUMMARY OF THE INVENTION

### <Problem the Invention is Intended to Solve>

However, in the above-mentioned X-ray inspection device, when articles are conveyed on a plurality of conveyors having different conveying speeds, an X-ray line sensor must be provided for each conveyor, which increases costs. Therefore, there is a demand for an X-ray inspection device that can inspect articles even when the articles are conveyed on a plurality of conveyors having different conveying speeds, without additional X-ray line sensors.

### <Means for Solving Problem>

An X-ray inspection device according to a first aspect comprises a first conveyor, a second conveyor, an X-ray irradiation unit, an X-ray line sensor, and a control unit. The first conveyor conveys a first article at a predetermined conveying speed. The second conveyor conveys a second article at a conveying speed different from that of the first conveyor while the first article is being conveyed on the first conveyor. The X-ray irradiation unit radiates X-rays toward the first article and the second article being conveyed by the first conveyor and the second conveyor. The X-ray line sensor detects the amount of X-rays transmitted through the first article and the second article at a timing based on the conveying speed of the first conveyor. The control unit generates a first X-ray image, which is an X-ray image of the first article, and a second X-ray image, which is an X-ray image of the second article, on the basis of the detection results of the X-ray line sensor, and inspects the first article and the second article on the basis of the first X-ray image and the second X-ray image, respectively. In addition, the control unit corrects an effect of a speed difference between the first conveyor and the second conveyor in the inspection of the second article based on the second X-ray image.

In the X-ray inspection device according to the first aspect, the X-ray line sensor detects the amount of X-rays transmitted through the first article and the second article at a timing based on the conveying speed of the first conveyor. For example, if the conveying speed of the first conveyor is higher than the conveying speed of the second conveyor, the image of the second article on the second conveyor will be extended in the conveying direction. However, because the conveying speeds of both conveyors are known, any effect from the speed difference in the inspection of the second article is preferably corrected. Therefore, there is no need to add additional X-ray line sensors.

An X-ray inspection device of a second aspect is the X-ray inspection device of the first aspect, wherein the first article is smaller in size than the second article.

The X-ray inspection device of the second aspect is suitable for a system in which the inspected second article is cut and conveyed as first articles on a first conveyor. For example, the X-ray inspection device can be applied to a system in which a block of meat is cut into small, fixed amounts.

An X-ray inspection device of a third aspect is the X-ray inspection device of the first or second aspect, wherein the conveying speed of the first conveyor is higher than the conveying speed of the second conveyor.

When the X-ray inspection device of the third aspect is incorporated into a system in which the inspected second article is cut and conveyed as first articles by the first conveyor, the quantity of first articles conveyed by the first conveyor will be greater than the quantity of second articles conveyed by the second conveyor. Therefore, it is reasonable for the conveying speed of the first conveyor to be higher than the conveying speed of the second conveyor.

An X-ray inspection device of a fourth aspect is the X-ray inspection device of any of the first to third aspects, wherein the control unit enlarges or shrinks the second X-ray image so that the second X-ray image is an X-ray image that would occur if the amount of X-rays transmitted through the second article were detected at a timing based on the conveying speed of the second conveyor.

In the X-ray inspection device of the fourth aspect, the second X-ray image is generated from the result of detecting the amount of X-rays transmitted through the second article at a timing based on the conveying speed of the first conveyor; therefore, the image shows the shape of the article stretched or shrunk depending on the ratio of the conveying speed of the second conveyor to the conveying speed of the first conveyor.

Therefore, by enlarging or shrinking the second X-ray image, it is possible to correct the second X-ray image to an X-ray image that would result if the amount of X-rays transmitted through the second article had been detected at a timing based on the conveying speed of the second conveyor. Thus, X-ray inspection of the first article and the second article is possible with a single X-ray line sensor.

An X-ray inspection device of a fifth aspect is the X-ray inspection device of any of the first to fourth aspects, wherein the X-ray inspection device further comprises a display unit to display the first X-ray image and the corrected second X-ray image.

When the X-ray inspection device of the fifth aspect is incorporated into a system in which an inspected second article is cut and conveyed as first articles on the first conveyor, it is possible to compare the articles before and after cutting, and whether the articles have been cut at the appropriate positions can be visually recognized.

An X-ray inspection device of a sixth aspect is the X-ray inspection device of any of the first to fifth aspects, wherein the X-ray inspection device further comprises a transmission unit. The transmission unit transmits predetermined information. The predetermined information includes a timing at which the first article or the second article is to be sorted, which is transmitted to a sorting device disposed downstream of the X-ray inspection device, or a timing at which the first article and/or the second article is to be cut, which is transmitted to a cutting device disposed downstream of the X-ray inspection device.

An X-ray inspection device of a seventh aspect is the X-ray inspection device of any of the first to fifth aspects, wherein the X-ray inspection device further comprises a transmission unit to transmit predetermined information. The conveying speed of the first conveyor is higher than the conveying speed of the second conveyor. Respective weights of the first article and the second article are inspected on the basis of the first X-ray image and the second X-ray image. The inspected second article is divided into a plurality of small articles of fixed amounts by a cutting device disposed downstream of the second conveyor. The small articles are conveyed as the first article by the first conveyor. The transmission unit identifies a cutting position when dividing the second article on the basis of the weights of the first article and the second article and the specified method of dividing the second article. Furthermore, the transmission unit transmits information related to the second article for which the cutting position has been identified and information related to the cutting position in association with each other to the cutting device.

When the X-ray inspection device of the seventh aspect is incorporated into a system in which an inspected second article is cut and conveyed as first articles on the first conveyor, the second article is precisely cut and divided into a plurality of small articles of fixed amounts on the basis of the information from the transmission unit.

An X-ray inspection device of an eighth aspect is the X-ray inspection device of the seventh aspect, wherein the X-ray inspection device further comprises a speed detection unit to detect an actual conveying speed of the second conveyor. The information related to the cutting position is cutting position information corrected on the basis of the actual conveying speed detected by the speed detection unit.

An X-ray inspection device of a ninth aspect is the X-ray inspection device of the eighth aspect, wherein the transmission unit corrects the second X-ray image on the basis of a ratio between a set conveying speed of the first conveyor and a set conveying speed of the second conveyor. Furthermore, the transmission unit corrects the corrected second X-ray image on the basis of a ratio between the set conveying speed of the second conveyor and the actual conveying speed detected by the speed detection unit.

An X-ray inspection device of a tenth aspect is the X-ray inspection device of any of the first to seventh aspects, wherein the X-ray inspection device further comprises a speed detection unit to detect an actual conveying speed of the second conveyor. The control unit corrects the second X-ray image on the basis of a ratio between a set conveying speed of the first conveyor and a set conveying speed of the second conveyor. Furthermore, the control unit corrects the corrected second X-ray image on the basis of a ratio between the set conveying speed of the second conveyor and the actual conveying speed detected by the speed detection unit.

An X-ray inspection device of an eleventh aspect is the X-ray inspection device of any of the first to tenth aspects, wherein a conveying direction of the first conveyor and a conveying direction of the second conveyor are opposite to each other.

When the X-ray inspection device of the eleventh aspect is incorporated into a system in which an inspected second article is cut and conveyed as first articles on the first conveyor, no time or effort will be expended in collecting the articles conveyed downstream of the second conveyor and flowing the articles to the first conveyor.

### <Effects of Invention>

In the X-ray inspection device according to the present invention, the X-ray line sensor detects the amount of X-rays transmitted through the first article and the second article at a timing based on the conveying speed of the first conveyor. If the conveying speed of the first conveyor is greater than the conveying speed of the second conveyor, the image of the second article on the second conveyor will be extended in the conveying direction. However, because the conveying speeds of both conveyors are known, the effect of the speed difference in the inspection of the second article is preferably corrected. Therefore, there is no need to add additional X-ray line sensors.

### BRIEF DESCRIPTION OF THE DRAWINGS

FIG. 1 is a schematic diagram of an X-ray inspection system including an X-ray inspection device according to one embodiment of the present invention;
FIG. 2 is a perspective view of the exterior of the X-ray inspection device;
FIG. 3 is a simplified diagram of the internal configuration of the X-ray inspection device;
FIG. 4 is a control block diagram of the X-ray inspection device;
FIG. 5 is a flowchart of a process related to weight estimation performed by a control unit;
FIG. 6 is a table comparing a second X-ray image of a second article when a second set speed and a first set speed are equal with a second X-ray image of the second article when the second set speed is half the first set speed;
FIG. 7 is a flowchart of a process related to identification of cutting positions by a PLC;
FIG. 8 is a chart comparing original image data, primary correction data, and secondary correction data for the second X-ray image; and
FIG. 9 is a configuration diagram of an X-ray inspection system including an X-ray inspection device according to a first modification.

### DESCRIPTION OF EMBODIMENTS

### (1) Configuration of X-ray inspection system 100

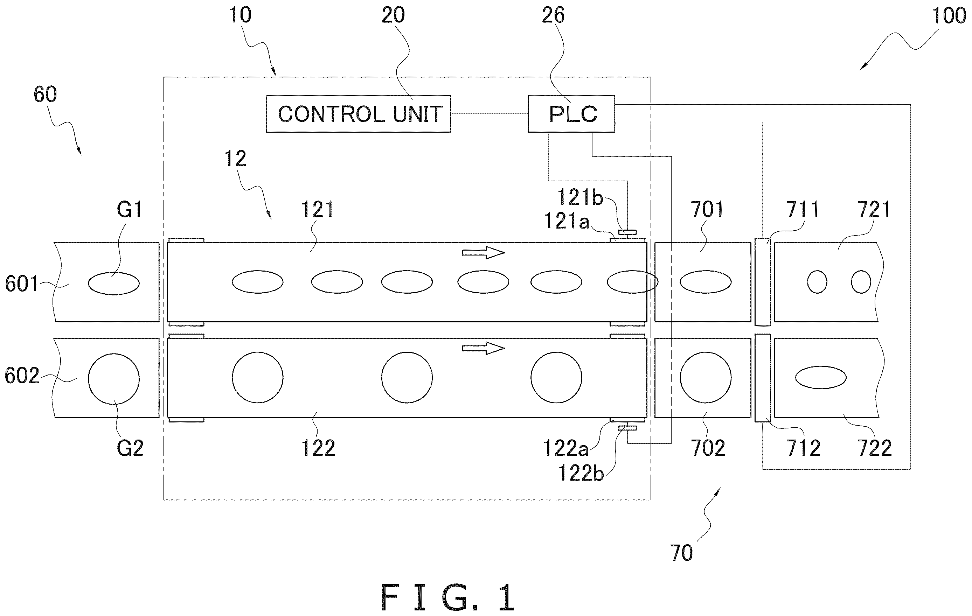

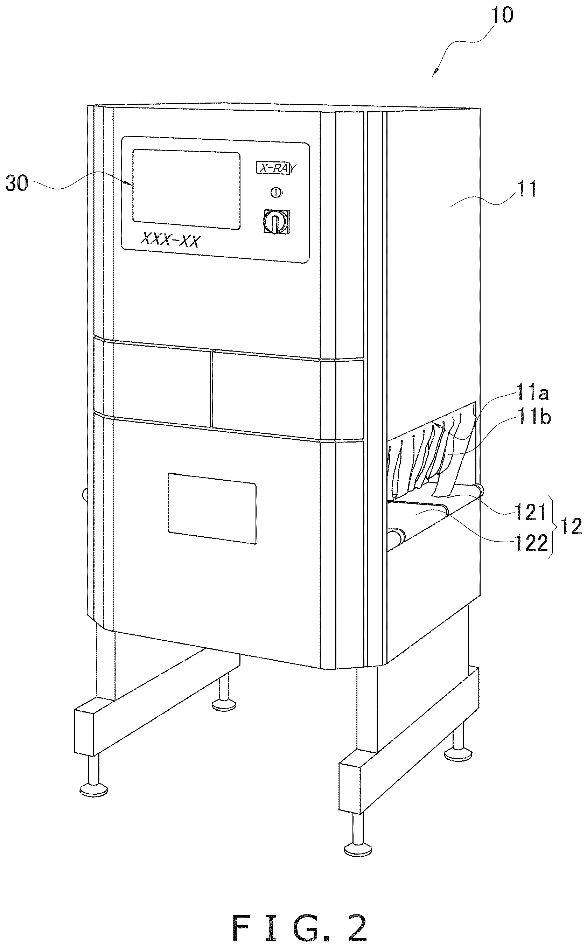

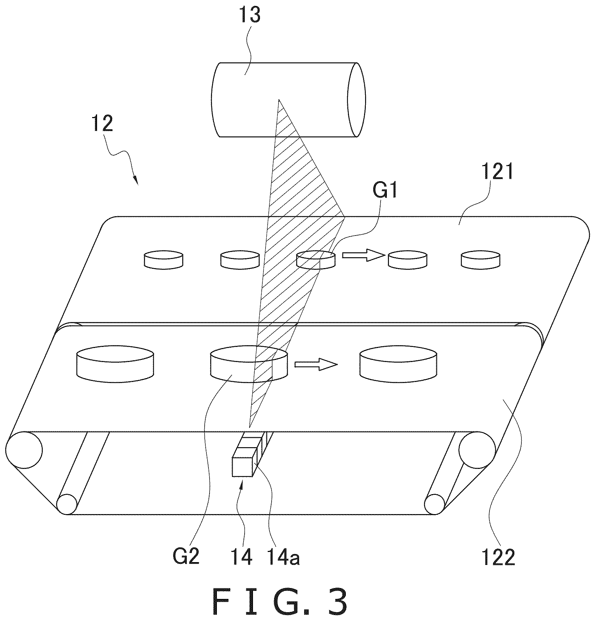

FIG. 1 is a schematic diagram of an X-ray inspection system 100 including an X-ray inspection device 10 according to one embodiment of the present invention. FIG. 2 is a perspective view of the exterior of the X-ray inspection device 10. Furthermore, FIG. 3 is a simplified diagram of the internal configuration of the X-ray inspection device 10.

As shown in FIG. 1, the X-ray inspection system 100 includes the X-ray inspection device 10, an article convey-in section 60 arranged upstream of the X-ray inspection device 10, and an article processing section 70 arranged downstream of the X-ray inspection device 10.

### (1-1) Article convey-in section 60

The article convey-in section 60 has a first convey-in conveyor 601 and a second convey-in conveyor 602. The first convey-in conveyor 601 continuously sends first articles G1 to a first conveyor 121 of the X-ray inspection device 10. The second convey-in conveyor 602 continuously sends second articles G2 to a second conveyor 122 of the X-ray inspection device 10. In the present embodiment, the first articles G1 and the second articles G2 are the same type of article.

### (1-2) X-ray inspection device 10

As shown in FIG. 3, the X-ray inspection device 10 radiates X-rays toward the first articles G1 and the second articles G2 that are continuously conveyed by the first conveyor 121 and the second conveyor 122, and estimates the weight of the first articles G1 and the second articles G2 on the basis of the amount of X-rays transmitted through the first articles G1 and the second articles G2. Details of the X-ray inspection device 10 shall be described in section "(2) Configuration of the X-ray inspection device 10."

### (1-3) Article processing section 70

The article processing section 70 performs predetermined processing on the first articles G1 and the second articles G2 for which inspection results from the X-ray inspection device 10 have been received.

In the present embodiment, the article processing section 70 has a first relay conveyor 701, a second relay conveyor 702, a first cutting device 711, a second cutting device 712, a first convey-out conveyor 721, and a second convey-out conveyor 722.

The first relay conveyor 701 conveys the first articles G1 of which weight has been estimated to the first cutting device 711. The first cutting device 711 cuts and divides the first articles G1 into small articles smaller than the first articles G1. The first convey-out conveyor 721 conveys out the small articles divided by the first cutting device 711 to a predetermined location.

The second relay conveyor 702 conveys the second articles G2 of which weight has been estimated to the second cutting device 712. The second cutting device 712 cuts and divides the second articles G2 into small articles smaller than the second articles G2. The second convey-out conveyor 722 conveys the small articles divided by the second cutting device 712 to a predetermined location.

In the present embodiment, the small articles divided by the second cutting device 712 are sent as first articles G1 to the first convey-in conveyor 601 of the article convey-in section 60.

### (2) Configuration of X-ray inspection device 10

In FIGS. 1, 2, and 3, the X-ray inspection device 10 includes a shield box 11, a conveying section 12, an X-ray irradiator 13, an X-ray line sensor 14, a monitor 30 with a touch panel function, and a control unit 20.

### (2-1) Shield box 11

The shield box 11 is a box to house the conveying section 12, the X-ray irradiator 13, the X-ray line sensor 14, and the control unit 20. The monitor 30 is located at a front upper part of the shield box 11.

The shield box 11 has openings 11a on both sides. The openings 11a are used to convey the first articles G1 and the second articles G2 into and out of the shield box 11.

Shielding curtains 11b are provided at the openings 11a. The shielding curtains 11b prevent X-rays inside the shield box 11 from leaking to the outside.

### (2-2) Conveying section 12

The conveying section 12 has the first conveyor 121 and the second conveyor 122. The first conveyor 121 and the second conveyor 122 are arranged so as to pass through the openings 11a of the shield box 11.

As shown in FIG. 1, the first conveyor 121 is driven by a first conveyor motor 121a. The second conveyor 122 is driven by a second conveyor motor 122a.

The first conveyor 121 receives the first articles G1 from the first convey-in conveyor 601, conveys the articles through the shield box 11, and delivers the articles to the first relay conveyor 701.

The second conveyor 122 receives the second articles G2 from the second convey-in conveyor 602, conveys the articles through the shield box 11, and delivers the articles to the second relay conveyor 702.

A speed at which the first articles G1 are conveyed by the first conveyor 121 is inputted by an operator and is referred to as a first set speed Vs1. Similarly, a speed at which the second articles G2 are conveyed by the second conveyor 122 is also inputted by the operator and is referred to as a second set speed Vs2.

In the present embodiment, the first set speed Vs1 is set higher than the second set speed Vs2; for example, the first set speed Vs1 of the first conveyor 121 is set to 1 m/sec, and the second set speed Vs2 of the second conveyor 122 is set to 0.5 m/sec. The first conveyor motor 121a and the second conveyor motor 122a are inverter-controlled on the basis of the first set speed Vs1 and the second set speed Vs2.

A first encoder 121b is attached to the first conveyor motor 121a, and a second encoder 122b is attached to the second conveyor motor 122a. The first encoder 121b detects a first actual speed Vr1, which is the actual conveying speed of the first conveyor 121. The second encoder 122b detects a second actual speed Vr2, which is the actual conveying speed of the second conveyor 122.

### (2-3) X-ray irradiator

As shown in FIG. 3, the X-ray irradiator 13 is disposed above the first conveyor 121 and the second conveyor 122, and irradiates the X-ray line sensor 14 with X-rays. The irradiation range fans out in a direction intersecting the conveying direction of the first conveyor 121 and the second conveyor 122.

### (2-4) X-ray line sensor

The X-ray line sensor 14 is disposed below conveying surfaces of the first conveyor 121 and the second conveyor 122, and the X-ray line sensor 14 is constituted of numerous X-ray detection elements 14a.

The X-ray detection elements 14a are horizontally disposed in a straight line perpendicular to the conveying direction of the first conveyor 121 and the second conveyor 122. The X-ray detection elements 14a detect X-rays transmitted through the first articles G1 and the second articles G2 and output X-ray transmission signals corresponding to the amounts of transmitted X-rays, and grayscale values of X-ray images are determined according to the X-ray transmission signals.

The X-ray line sensor 14 detects the amount of X-rays transmitted through the first articles G1 and the second articles G2 at a timing based on the first set speed Vs1 of the first conveyor 121.

### (2-5) Monitor 30

The monitor 30 is a liquid crystal display. The monitor 30 displays a screen prompting the operator to input inspection parameters and the like required during inspection.

### (2-6) Control unit 20

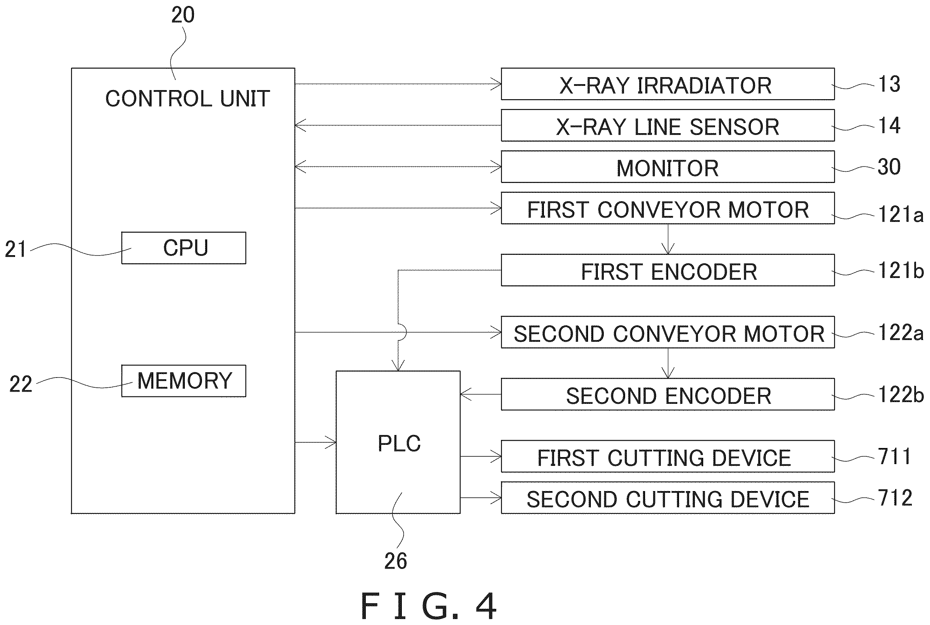

FIG. 4 is a control block diagram of the X-ray inspection device 10. In FIG. 4, the control unit 20 includes a CPU 21 and a memory 22. The control unit 20 is connected to the X-ray irradiator 13, the X-ray line sensor 14, the monitor 30, the first conveyor motor 121a, the second conveyor motor 122a, and a programmable logic controller (PLC) 26. The control unit 20 estimates the weights of the first articles G1 and the second articles G2 and/or inspects the articles for defects such as the presence of foreign objects.

### (2-7) PLC 26

The PLC 26 is connected to the first encoder 121b, the second encoder 122b, the first cutting device 711, and the second cutting device 712. The PLC 26 identifies positions where the first articles G1 and the second articles G2 are to be cut. The PLC 26 receives, from the control unit 20, "conveying direction length information" for the first articles G1 and the second articles G2 and "information related to cutting positions" for the first articles G1 and the second articles G2.

In addition, the PLC 26 identifies the positions where the first articles G1 and the second articles G2 are to be cut from the received information and ratios between the set speeds and actual speeds of the conveyors.

Furthermore, the PLC 26 has a transmission function, and transmits information related to the first articles G1 and the second articles G2 for which the cutting positions have been identified and information related to the cutting positions of the first articles G1 and the second articles G2 in association with each other to the first cutting device 711 and the second cutting device 712.

### (3) Actions of control unit 20 of X-ray inspection device 10

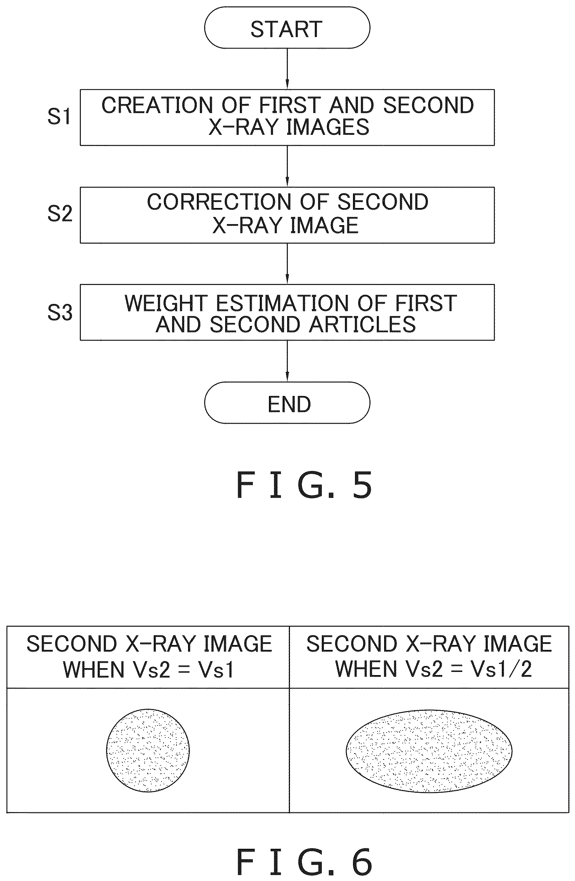

FIG. 5 is a flowchart of a process related to weight estimation performed by the control unit 20.

### (Step S1)

In step S1, the control unit 20 creates a first X-ray image that is an X-ray image of a first article G1 and a second X-ray image that is an X-ray image of a second article G2 on the basis of the amount of X-rays transmitted through the first article G1 and the second article G2 detected by the X-ray line sensor 14.

Furthermore, the control unit 20 transmits the "conveying direction length information" of the first article G1 and the second article G2 and the "information related to the cutting positions" of the first article G1 and the second article G2 to the PLC 26 and causes the information to be stored in the memory 22.

### (Step S2)

In step S2, the control unit 20 corrects the second X-ray image. Image data for the second X-ray image is the result of detecting the amount of X-rays transmitted through the second article G2 at a timing based on the conveying speed of the first conveyor 121, and is data of an image extending in the conveying direction. Regardless of whether an image is a first X-ray image or a second X-ray image, image data before correction is referred to as "original image data."

The control unit 20 knows in advance the first set speed Vs1, which is the conveying speed set for the first conveyor 121, and the second set speed Vs2, which is the conveying speed set for the second conveyor 122, and therefore corrects the original image data of the second X-ray image on the basis of the ratio Vs2/Vs1 of the second set speed Vs2 to the first set speed Vs1.

For a more specific explanation, refer to FIG. 6. FIG. 6 is a table comparing a second X-ray image of a second article G2 when the second set speed Vs2 and the first set speed Vs1 are equal to a second X-ray image of a second article G2 when the second set speed Vs2 is half the first set speed Vs1.

In FIG. 6, the image data of the second X-ray image is the result of detecting the amount of X-rays transmitted through the second article G2 at a timing based on the conveying speed of the first conveyor 121, and because the second set speed Vs2 is half the first set speed Vs1, the length of the second X-ray image in the conveying direction of the second article G2 is twice as long, as shown in the right side of FIG. 6. Therefore, the original image data of the second X-ray image is preferably corrected so that the length in the conveying direction is halved. The X-ray image of the second article G2 obtained by correcting the original image data is referred to as the "corrected second X-ray image."

### (Step S3)

In step S3, the control unit 20 estimates the weight of the first article G1 from the first X-ray image and estimates the weight of the second article G2 from the corrected second X-ray image.

The control unit 20 estimates a weight value of an article by utilizing the property that the thicker a substance is in the direction of X-ray irradiation in an X-ray image, the darker the image will appear.

As described above, in the X-ray inspection device 10 according to the present embodiment, the X-ray line sensor 14 detects the amount of X-rays transmitted through the first article G1 and the second article G2 at a timing based on the first set speed Vs1 of the first conveyor 121. Therefore, when the first set speed Vs1 of the first conveyor 121 is higher than the second set speed Vs2 of the second conveyor 122, the image of the second article G2 on the second conveyor 122 will be an image that is extended in the conveying direction.

However, since the conveying speeds of both conveyors are known, the effect of the speed difference can be corrected by multiplying the length of the original image data of the second X-ray image in the conveying direction by the ratio Vs2/Vs1. therefore, there is no need to add additional X-ray line sensors 14.

### (4) Actions of PLC 26 of X-ray inspection device 10

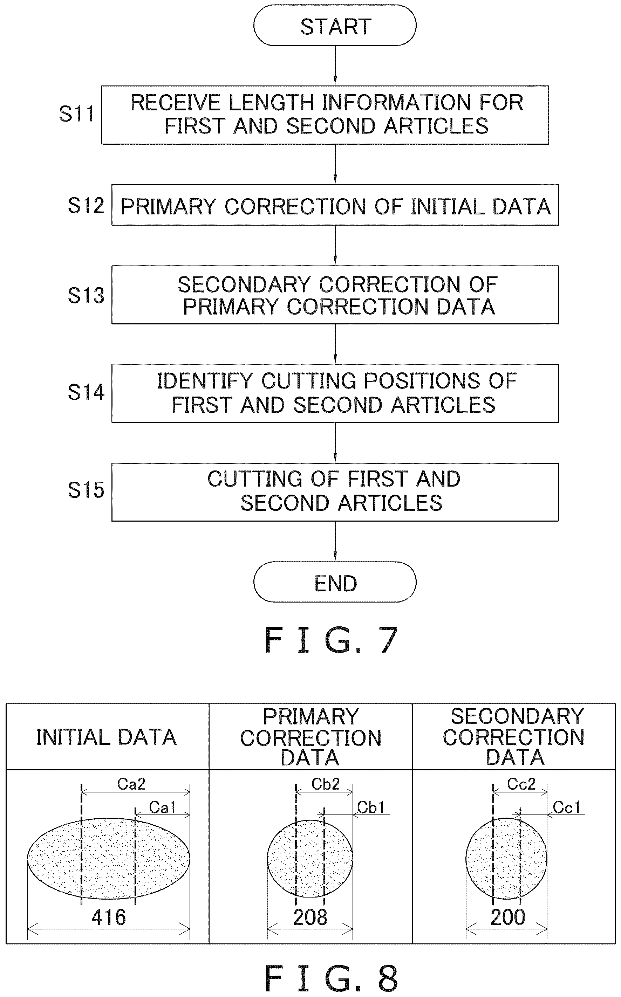

FIG. 7 is a flowchart of a process relating to identification of the cutting positions by the PLC 26.

### (Step S11)

In step S11, the PLC 26 receives, from the control unit 20, "conveying direction length information" for the first article G1 and the second article G2 and "information related to cutting positions" for the first article G1 and the second article G2.

The original image data of the second X-ray image, which is the basis for the "conveying direction length information" of the second article G2, is the result of detecting the amount of X-rays transmitted through the second article G2 at a timing based on the conveying speed of the first conveyor 121, and the length of the second article G2 is the length extended in the conveying direction. This extended length is referred to as "initial data."

### (Step S12)

In step S12, the PLC 26 multiplies the length of the second article G2 in the conveying direction by the ratio Vs2/Vs1 of the second set speed Vs2 to the first set speed Vs1, and corrects the initial data. This is referred to as primary correction. The data obtained by performing primary correction on the initial data is referred to as "primary correction data."

The above correction shall now be described in detail. For example, if the first set speed Vs1 is twice the second set speed Vs2, the image displayed on the basis of the original image data of the second X-ray image will be twice as long in the conveying direction as the actual image, and an inspection weight will also be twice the actual weight. Therefore, the PLC 26 multiplies the length of the second article G2 in the conveying direction and the inspection weight by 1/2 to obtain primary correction data. The length of the first article G1 in the conveying direction is unaffected by the speed difference between the first conveyor 121 and the second conveyor 122; therefore, the length is multiplied by a ratio of 1 to perform a primary correction, and this is considered to be the primary correction data.

### (Step S13)

In step S13, the PLC 26 corrects the primary correction data on the basis of the first actual speed Vr1 and the second actual speed Vr2 detected by the first encoder 121b and the second encoder 122b.

For example, the primary correction data for the second article G2 is corrected on the basis of the ratio Vs2/Vs1 of the second set speed Vs2 to the first set speed Vs1, but the second actual speed Vr2 of the second conveyor 122 is not necessarily the second set speed Vs2. Therefore, the PLC 26 further corrects the primary correction data on the basis of the ratio Vr2/Vs2 of the second actual speed Vr2 to the second set speed Vs2. The data obtained by correcting the primary correction data is referred to as "secondary correction data."

For example, when the second actual speed Vr2 of the second conveyor 122 is 4% lower than the second set speed Vs2, the length in the conveying direction based on the primary correction data for the second article G2 and the inspection weight are multiplied by 0.96 to obtain secondary correction data.

Similarly, for the first article G1, the first actual speed Vr1 of the first conveyor 121 is not necessarily the first set speed Vs1. Therefore, the PLC 26 further performs a secondary correction on the primary correction data on the basis of the ratio Vr1/Vs1 of the first actual speed Vr1 to the first set speed Vs1, to obtain secondary correction data.

For a more specific explanation, refer to FIG. 8. FIG. 8 is a chart comparing initial data, primary correction data, and secondary correction data for a second article G2.

In FIG. 8, it is assumed that the actual second article G2 is a circular object having a diameter of 200 mm as seen in plan view. It is also assumed that the first set speed Vs1 of the first conveyor 121 is 1 m/sec, and the second set speed Vs2 of the second conveyor 122 is 0.5 m/sec. Furthermore, it is assumed that the second actual speed Vr2 of the second conveyor 122 is 0.48 m/sec. The original image data of the second X-ray image, which is the basis for the "conveying direction length information" of the second article G2, is obtained by detecting the amount of X-rays transmitted through the second article G2 at a timing based on the first set speed Vs1 (1 m/sec) of the first conveyor 121. Therefore, if the conveying speed of the second conveyor 122 is the second set speed Vs2 = 0.5 m/sec, the length of the second article G2 in the conveying direction should be 400 mm.

However, because the second actual speed Vr2 = 0.48 m/sec, the length of the second article G2 in the conveying direction is 416 mm. If the second article G2 were cut at a first imaginary cutting position Ca1 and a second imaginary cutting position Ca2 in the initial data, there would be no cutting at the second imaginary cutting position Ca2.

Therefore, as a primary correction, the length of 416 mm is multiplied by the ratio of the second set speed Vs2 to the first set speed Vs1, Vs2/Vs1 = 1/2, to correct the length to 208 mm. However, even if the second article G2 is cut at a first primary corrected cutting position Cb1 and a second primary corrected cutting position Cb2 after the primary correction, it will not be possible to precisely divide the article into fixed amounts.

Next, as a secondary correction, the length of 208 mm is multiplied by the ratio of the second actual speed Vr2 to the second set speed Vs2, Vr2/Vs2 = 0.96, to correct the length to 200 mm. By cutting the second article G2 at a first secondary corrected cutting position Cc1 and a second secondary corrected cutting position Cc2 after the secondary correction, the article can be precisely divided into fixed amounts.

In the present embodiment, a side end of the second article G2 in the conveying direction is set as the starting point of the cutting positions.

### (Step S14)

In step S14, the PLC 26 identifies cutting positions for dividing the first article G1 and the second article G2 into fixed amounts on the basis of the secondary correction data. It is assumed that the method of dividing the first article G1 and the second article G2 is set in advance.

### (Step S15)

In step S15, the PLC 26 cuts the first article G1 at the cutting position identified by the PLC 26 via the first cutting device 711, and cuts the second article G2 at the cutting position identified by the PLC 26 via the second cutting device 712.

As described above, in the X-ray inspection device 10 according to the present embodiment, the PLC 26 identifies the cutting positions of the first article G1 and the second article G2 on the basis of the first set speed Vs1, the second set speed Vs2, the first actual speed Vr1, and the second actual speed Vr2, and the first article G1 and the second article G2 can therefore be divided with high precision.

In the present embodiment, the second article G2 is cut and divided into small articles by the second cutting device 712, which are then collected and placed on the first convey-in conveyor 601 as first articles G1. In addition, after the first articles G1 are cut and divided into small articles by the first cutting device 711, the small articles are packaged in predetermined quantities to become commercial products.

### (5) Characteristics

(5-1) In the X-ray inspection device 10, the X-ray line sensor 14 detects the amount of X-rays transmitted through the first articles G1 and the second articles G2 at a timing based on the conveying speed of the first conveyor 121. Because the conveying speeds of the first conveyor 121 and the second conveyor 122 are known, the effect of the speed difference in the inspection of the second articles G2 can be corrected. Therefore, there is no need to add additional X-ray line sensors 14.
(5-2) Since the first articles G1 are smaller in size than the second articles G2, an inspected second article G2 can be divided into a plurality of items and conveyed as first articles G1 on the first conveyor. This can be applied to a process of cutting a block of meat into small, fixed amounts.
(5-3) The conveying speed of the first conveyor 121 is higher than the conveying speed of the second conveyor 122. When an inspected second article G2 is divided into a plurality of items and conveyed as first articles G1 by the first conveyor 121, the quantity of first articles G1 conveyed by the first conveyor 121 will be greater than the quantity of second articles G2 conveyed by the second conveyor 122. Therefore, it is reasonable for the conveying speed of the first conveyor 121 to be higher than the conveying speed of the second conveyor 122.
(5-4) In the X-ray inspection device 10, the second X-ray image is generated from the result of detecting the amount of X-rays transmitted through the second article G2 at a timing based on the conveying speed of the first conveyor 121; therefore, the image shows the shape of the article stretched or shrunk depending on the ratio of the speed of the second conveyor 122 to that of the first conveyor 121.

Therefore, by enlarging or shrinking the second X-ray image, it is possible to correct the second X-ray image to an X-ray image that would result if the amount of X-rays transmitted through the second article G2 had been detected at a timing based on the conveying speed of the second conveyor 122. Thus, X-ray inspection of first articles G1 and second articles G2 is possible with a single X-ray line sensor 14.
(5-5) In the X-ray inspection device 10, when an inspected second article G2 is divided into a plurality of items and conveyed as first articles G1 on the first conveyor 121, the monitor 30 makes it possible to compare the articles before and after cutting, and to visually recognize whether the articles have been cut at the appropriate positions.
(5-6) The PLC 26 transmits predetermined information to the first cutting device 711 and the second cutting device 712 or a sorting device 76, which are arranged downstream of the X-ray inspection device 10. The predetermined information includes the timing at which the first cutting device 711 cuts first articles G1, the timing at which the second cutting device 712 cuts second articles G2, the timing when the sorting device 76 sorts first articles G1 or second articles G2, etc.
(5-7) The PLC 26 identifies the cutting positions for when the second articles G2 are divided on the basis of the weights of the first articles G1 and the second articles G2 and the specified method for dividing the second articles G2, and transmits information about the second articles G2 for which the cutting positions have been identified in association with information about the cutting positions to the second cutting device 712. On the basis of the information from the PLC 26, the second articles G2 can be precisely cut and divided into a plurality of small articles of a fixed amount.
(5-8) The X-ray inspection device 10 is provided with a second encoder 122b to detect the actual conveying speed of the second conveyor 122. The information regarding the cutting positions is cutting position information corrected on the basis of the actual conveying speed detected by the second encoder 122b.
(5-9) The PLC 26 corrects the lengths of the second articles G2 in the conveying direction on the basis of the ratio between the first set speed Vs1 of the first conveyor 121 and the second set speed Vs2 of the second conveyor 122, and further corrects the corrected lengths in the conveying direction on the basis of the ratio between the second set speed Vs2 of the second conveyor 122 and a second actual speed Vr2, which is the actual conveying speed detected by the second encoder 122b. However, the above correction may be performed by the control unit 20 instead of the PLC 26.

### (6) Modifications

### (6-1) First modification

In the above embodiment, the conveying directions of the first conveyor 121 and the second conveyor 122 are the same, but the conveying directions of the first conveyor 121 and the second conveyor 122 may be opposite to each other.

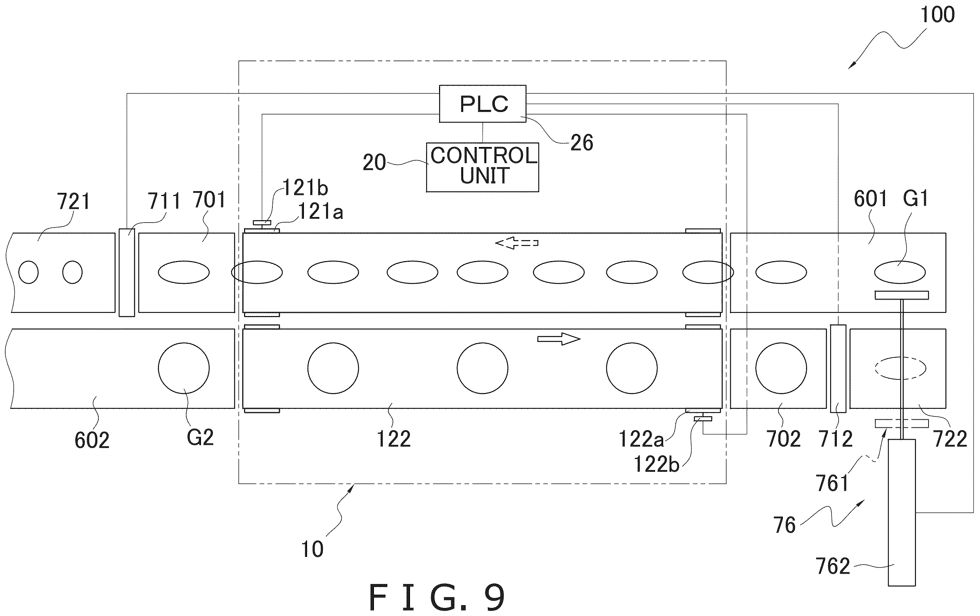

FIG. 9 is a configuration diagram of the X-ray inspection system 100 including the X-ray inspection device 10 according to a first modification. In FIG. 9, second articles G2 on the second conveyor 122 are conveyed in the direction of the solid-line arrow as seen from the front of the drawing.

First articles G1 on the first conveyor 121 are conveyed in the direction of the broken-line arrow, which is opposite to the conveying direction of the second conveyor 122. The first set speed Vs1 of the first conveyor 121 is twice the second set speed Vs2 of the second conveyor 122.

The first cutting device 711 and the second cutting device 712 are disposed downstream in the conveying direction of the first conveyor 121 and the second conveyor 122, respectively.

In the first modification, as in the above embodiment, image data for the second X-ray image is the result of detecting the amount of X-rays transmitted through the second article G2 at a timing based on the conveying speed of the first conveyor 121.

Therefore, the PLC 26 corrects the length (initial data) extending in the conveying direction by primary and secondary corrections to identify the cutting positions of the second article G2. The second cutting device 712 cuts the second article G2 at the cutting positions identified by the PLC 26. The second article G2 cut by the second cutting device 712 is an article that passed the weight inspection by the X-ray inspection device 10 without the inclusion of any foreign objects or the like having been detected therein.

In the first modification, a sorting device 76 is provided downstream of the second cutting device 712. The sorting device 76 pushes the small articles cut and divided by the second cutting device 712 as first articles G1 to the first convey-in conveyor 601 located upstream of the first conveyor 121 in the conveying direction. The sorting device 76 has a pushing member 761 to push out the small articles, and an air cylinder 762 to cause the pushing member 761 to perform a pushing action and a pulling action. However, second articles that have failed the weight inspection by the X-ray inspection device 10 or that have had the presence of foreign objects or the like detected therein are not cut by the second cutting device 712 and not pushed out by the sorting device 76, but are conveyed and discharged by the second convey-out conveyor 722. The sorting timing is preferably determined on the basis of the second X-ray image after correction.

As described above, in the X-ray inspection system 100, small articles divided from the second articles G2 are automatically pushed out as first articles G1 to the upstream side of the first conveyor 121, thus saving the time and effort of collecting the articles conveyed downstream of the second conveyor 122 and flowing the articles to the first conveyor 121.

The first articles G1 are products that are cut and divided into small articles by the first cutting device 711 and then packaged into predetermined quantities. In the first modification, it is not necessary for the sorting device 76 to be disposed downstream of the second cutting device 712. For example, when the articles are raw meat, it is possible to sort not only portions of the meat after cutting, but also whole meat immediately after inspection before cutting. In addition, if the articles include rod-form metal foreign objects over the entirety of the conveying direction of the articles, cutting the object first increases the risk of chipping the blade and turning the blade chips into foreign objects that may contaminate the articles. In such cases, the sorting device is placed downstream of the X-ray inspection device 10 and upstream of the second cutting device 712.

### (6-2) Second modification

In the above embodiment, the weight of first articles G1 on the first conveyor 121 and of second articles G2 on the second conveyor 122 is inspected, but this method is not provided by way of limitation. For example, the weight of the articles may be inspected as a primary inspection on the first conveyor 121, which conveys the articles at high speed, and any defectives may be re-inspected as a second inspection on the second conveyor 122, which conveys the articles at low speed.

### (6-3) Third modification

In the second modification described above, the primary inspection and secondary inspection are combined into one inspection, but the inspection conditions may be different for the primary inspection and the secondary inspection. For example, the inspection conditions may be set so that the primary inspection is an inspection for the presence of metal foreign objects on the first conveyor 121, which conveys the articles at high speed, and the secondary inspection checks for the presence of resin foreign objects on the second conveyor 122, which conveys the articles at low speed.

As should be apparent, the inspection may be performed under such conditions that the presence of resin foreign objects is inspected on the first conveyor 121, and the presence of metal foreign objects is inspected on the second conveyor 122. Furthermore, the inspection is not limited to inspecting for the presence of foreign objects; an inspection for the presence of cavities, presence of jamming, number, shape, etc., may also be included.

### (6-4) Fourth modification

In the third modification described above, the primary inspection is performed on the first conveyor 121, which conveys the articles at high speed, and the secondary inspection is performed on the second conveyor 122, which conveys the articles at low speed, but the primary inspection may be performed on the second conveyor 122, which conveys the articles at low speed, and the secondary inspection may be performed on the first conveyor 121, which conveys the articles at high speed. For example, in the inspection of raw meat, the meat is carefully inspected for the inclusion of metal foreign objects on the second conveyor 122, which conveys the articles at low speed, and inspected for the inclusion of foreign objects such as cartilage, which should be removed but will not cause any problems if present, on the first conveyor 121, which conveys the articles at high speed.

A configuration in which a sorting device is disposed downstream of the second conveyor 122, which conveys the articles at low speed, and a cutting device is disposed downstream of the first conveyor 121, which conveys the articles at high speed, may also be used. If a metal foreign object is included at the cutting position, there is a risk that the blade will come into contact with the metal foreign object and chip. Therefore, the articles are first inspected on the second conveyor, which conveys the articles at low speed, to determine whether or not a cut should be made. Furthermore, if a metal foreign object is found, the cutting position may be changed to avoid the object. As a result, the blade is prevented from deteriorating.

If 100g of raw meat is to be divided into 30g portions, 10g of leftover meat will be conveyed downstream. Conversely, if a 100g article is to be divided into 80g portions, 20g of leftover meat will be produced. Therefore, a decision is made as to whether or not the raw meat should be cut on the second conveyor 122, which conveys the articles at low speed, and only the raw meat to be cut is conveyed to the first conveyor 121, which conveys the articles at high speed.

Alternatively, a decision is made as to whether or not a cutting process that does not produce leftover meat should be performed, and if 100g of raw meat is to be cut into 30g portions, the raw meat will be divided into 33g, 33g, and 34g portions.

### (6-5) Fifth modification

In the above embodiment, the conveying section 12 was assumed to include only the first conveyor 121 and the second conveyor 122, but one more conveyor may be added as a third conveyor.

### (6-6) Sixth modification

The control unit 20 may store in the memory 22 a table of correction coefficients corresponding to the speed difference or speed ratio between the first conveyor 121 and the second conveyor 122, and may use the table during correction.

### (6-7) Seventh modification

The primary and secondary corrections for correcting the effect caused by the speed difference between the first conveyor 121 and the second conveyor 122 may be performed by directly processing the original image data, or by enlarging or shrinking the displayed image.

### (6-8) Eighth modification

The X-ray inspection device 10 may be applied to a system in which articles of the same type and shape are conveyed by a first conveyor 121 and a second conveyor 122, and a first cutting device 711 and a second cutting device 712 are disposed downstream of the first conveyor 121 and the second conveyor 122, respectively. In this case, the small articles produced by cutting the articles with the first cutting device 711 will be smaller than the small articles produced by cutting the articles with the second cutting device 712. Because the conveying speed of the first conveyor 121 is higher than the conveying speed of the second conveyor 122, the first cutting device 711 operates higher speed than the second cutting device 712.

### REFERENCE SIGNS LIST

- 10: X-ray inspection device
- 13: X-ray irradiator (X-ray irradiation unit)
- 14: X-ray line sensor
- 20: Control unit
- 26: PLC (transmission unit)
- 30: Monitor (display unit)
- 121: First conveyor
- 122: Second conveyor
- 711: First cutting device
- 712: Second cutting device
- G1: First article
- G2: Second article

### PRIOR ART LITERATURE

### PATENT LITERATURE

[Patent Literature 1] Japanese Laid-open Patent Publication No. 2003-139723

## Claims

1. An X-ray inspection device comprising:
a first conveyor configured to convey a first article at a predetermined conveying speed;
a second conveyor configured to convey a second article at a conveying speed different from that of the first conveyor while the first article is being conveyed on the first conveyor;
an X-ray irradiation unit configured to radiate X-rays toward the first article and the second article being conveyed by the first conveyor and the second conveyor;
an X-ray line sensor configured to detect the amount of X-rays transmitted through the first article and the second article at a timing based on the conveying speed of the first conveyor; and
a control unit configured to generate a first X-ray image, which is an X-ray image of the first article, and a second X-ray image, which is an X-ray image of the second article, on the basis of the detection results from the X-ray line sensor, and to inspect the first article and the second article on the basis of the first X-ray image and the second X-ray image, respectively,
the control unit being configured to correct any effect of a speed difference between the first conveyor and the second conveyor in the inspection of the second article based on the second X-ray image.

2. The X-ray inspection device according to claim 1, wherein
the first article is smaller in size than the second article.

3. The X-ray inspection device according to claim 1 or claim 2, wherein
the conveying speed of the first conveyor is higher than the conveying speed of the second conveyor.

4. The X-ray inspection device according to any one of claims 1 to 3, wherein
the control unit is further configured to enlarge or shrink the second X-ray image so as to yield an X-ray image that would result if the amount of X-rays transmitted through the second article were detected at a timing based on the conveying speed of the second conveyor.

5. The X-ray inspection device according to any one of claims 1 to 4, wherein
the X-ray inspection device further comprises a display unit to display the first X-ray image and the corrected second X-ray image.

6. The X-ray inspection device according to any one of claims 1 to 5, wherein:
the X-ray inspection device further comprises a transmission unit to transmit predetermined information; and
the predetermined information includes
a timing at which the first article or the second article is to be sorted, which is transmitted to a sorting device disposed downstream of the X-ray inspection device with respect to a conveying direction of the first conveyor or the second conveyor, or
a timing at which the first article and/or the second article is to be cut, which is transmitted to a cutting device disposed downstream of the X-ray inspection device with respect to the conveying direction.

7. The X-ray inspection device according to any one of claims 1 to 6, wherein:
the X-ray inspection device further comprises a transmission unit to transmit predetermined information; and
when the conveying speed of the first conveyor is higher than the conveying speed of the second conveyor,
weights of each the first article and the second article are inspected on the basis of the first X-ray image and the second X-ray image, respectively, the inspected second article is divided into a plurality of small articles of fixed amounts by a cutting device disposed downstream of the second conveyor with respect to the conveying direction, and the small articles are conveyed as the first article by the first conveyor, and
the transmission unit is configured to identify a cutting position for when the second article is divided on the basis of the weights of each of the first article and the second article and the specified method of dividing the second article, and transmits, to the cutting device, information related to the second article for which the cutting position has been identified and information related to the cutting position in association with each other.

8. The X-ray inspection device according to claim 7, wherein
the X-ray inspection device further comprises a speed detection unit to detect an actual conveying speed of the second conveyor, and
the information related to the cutting position is cutting position information corrected on the basis of the actual conveying speed detected by the speed detection unit.

9. The X-ray inspection device according to claim 8, wherein
the transmission unit is configured to correct the second X-ray image on the basis of a ratio between a set conveying speed of the first conveyor and a set conveying speed of the second conveyor, and further correct the corrected second X-ray image on the basis of a ratio between the set conveying speed of the second conveyor and the actual conveying speed detected by the speed detection unit.

10. The X-ray inspection device according to any one of claims 1 to 7, wherein:
the X-ray inspection device further comprises a speed detection unit to detect an actual conveying speed of the second conveyor; and
the control unit is configured to correct the second X-ray image on the basis of a ratio between a set conveying speed of the first conveyor and a set conveying speed of the second conveyor, and further correct the corrected second X-ray image on the basis of a ratio between the set conveying speed of the second conveyor and the actual conveying speed detected by the speed detection unit.

11. The X-ray inspection device according to any one of claims 1 to 10, wherein
a conveying direction of the first conveyor and a conveying direction of the second conveyor are opposite to each other.
